# EUROPEAN PATENT SPECIFICATION

(11) **EP 1 197 222 B1**
(45) Date of publication and mention of the grant of the patent: **01.08.2007**
(21) Application number: 01128974.1
(22) Date of filing: 16.12.1992
(51) Int. Cl.: A61K 38/27, A61K 47/22, A61K 47/10, A61K 47/26, C07K 14/61

(54) **A stabilized pharmaceutical formulation comprising growth hormone and histidine**
Stabilisierte pharmazeutische Formulierung, die Wachstumshormon und Histidin enthält
Formulation pharmaceutique stabilisée comportant une hormone de croissance et de l'histidine

(30) Priority: 20.12.1991 DK 204691; 10.11.1992 DK 136492
(43) Date of publication of application: 17.04.2002
(62) Divisional of application: 93902089.7
(73) Proprietor: NOVO NORDISK A/S, 2880 Bagsvaerd (DK)
(72) Inventor: Sorensen, Hans Holmegaard, DK-2830 Virum (DK); Skriver, Lars, DK-2930 Vedbaek (DK); Hoelgaard, Annie Rassing, DK-2840 Holte (DK)

(56) References cited:
- EP-A- 0 374 120
- WO-A-92/00998
- US-A- 4 816 568
- US-A- 4 917 685

## Description

### FIELD OF THE INVENTION

The present invention relates to a stabilized pharmaceutical formulation comprising a human growth hormone derivative.

### BACKGROUND OF THE INVENTION

The growth hormones (GH) from man and from the common domestic animals are proteins of approximately 191 amino acids, synthesized and secreted from the anterior lope of the pituitary gland. Human growth hormone consists of 191 amino acids.

Growth hormone is a key hormone involved in the regulation of not only somatic growth, but also in the regulation of metabolism of proteins, carbohydrates and lipids. The major effect of growth hormone is to promote growth.

The organ systems affected by growth hormone include the skeleton, connective tissue, muscles, and viscera such as liver, intestine, and kidneys.

Until the development of the recombinant technology and the cloning of the growth hormone gene now giving rise to production of e.g. human growth hormone (hGH) and Met-hGH in industrial scale, human growth hormone could only be obtained by extraction from the pituitary glands of human cadavers. The very limited supplies of growth hormone restricted the use thereof to longitudinal growth promotion in childhood and puberty for treatment of dwarfism, even though it has been proposed for inter alia treatment of short stature (due to growth hormone deficiency, normal short stature and Turner syndrom), growth hormone deficiency in adults, infertility, treatment of burns, wound healing, dystrophy, bone knitting, osteoporosis, diffuse gastric bleeding, and pseudoarthrosis.

Furthermore, growth hormone has been proposed for increasing the rate of growth of domestic animals or for decreasing the proportion of fat in animals to be slaughtered for human consumption.

Pharmaceutical preparations of growth hormone tend to be unstable. Degradation products such as deamidated or sulfoxy-dated products and dimer or polymer forms are generated - especially in solutions of growth hormone.

The predominant degradation reactions of hGH are 1) deamidation by direct hydrolysis or via a cyclic succinimide inter-meadiate to form various amounts of L-asp-hGH, L-iso-asp-hGH, D-asp-hGH, and D-iso-asp-hGH (ref 1 , 2) oxidation of the methionine residues in positions 14 and 125 (ref 4-9, and 3) cleavage of peptide bonds.

Deamidation especially takes place at the Asn in position 149.

hGH is rather easily oxidized in positions 14 and 125, especially in solution (4-8).

The oxidation of hGH in solution forming sulfoxides is normally due to the oxygen dissolved in the preparation. The solubility of oxygen in distilled water is about 200 µM (9). As the concentration of hGH in a preparation comprising 4 IU/ml is 1.3 mg/ml corresponding to 60 nM hGH, oxygen will, at normal storing conditions, be present in an excess of about 3000 times the stoichiometric amount for oxidation of hGH. It is not feasible to try to solve the problem by degassing of buffers before tapping and packing the preparations.

At present, it is not believed that these degradation products should have toxic or altered biological activity or receptor binding properties, but there is indication to the effect that the conformation stability of the sulfoxides is reduced as compared to native hGH.

For the development of a stable, dissolved preparation comprising hGH it is of importance to know the rate of formation of sulfoxides as well as means to control the oxidation.

The kinetics of degradation depend on temperature, pH and various additives or adjuvants in the hGH formulation.

Due to the instability, growth hormone is, at present, lyophilized and stored in the lyophilized form at 4°C until it is reconstituted for use in order to minimize the degradation.

The lyophilized pharmaceutical preparations comprising hGH are, at present, reconstituted by the patient and then stored at a low temperature, often at about 4°C in the refrigerator as a solution during the period of use of up to 14 days, during which some degradation will take place.

Furthermore, the process of reconstitution of the lyophilized growth hormone tends to provide difficulties for the patient.

Thus, it is at present preferred to reconstitute the growth hormone as late as possible before use and to store and ship the preparation in a lyophilized state. The chain from the manufactorer to the pharmacy is apt for handling the preparations at a controlled low temperature of e.g. 4°C which allows for a long shelf life of up to two years.

However, the extended use of pen systems for self-medication and the expanded field of use calls for a preparation which is stable for a sufficient long time with the end user under conditions where "sufficient" cooling is not always available.

Preferably, a preparation should be stable with the end user in a lyophilized state for about one month and additionally for one month in a reconstituted state in a pen device for the intended period of use of a cartridge.

Thus, there is a need for more stable preparations of growth hormone being stable in a lyophilized state at a relative high temperature for a period and additionally for a period of use at a relatively high temperature in solution. Such stabilization is of very great importance when moving the administration of the growth hormone from clinics to the homes of the individuals to be treated where optimal storage may not be available as indicated above.

Furthermore, the shift in pattern of administration of growth hormone to the use of pen devices calls for a stable dissolved preparation comprising growth hormone in order to facilitate the handling to be performed by the patient. A stable dissolved preparation comprising growth hormone may be produced ready to use in the form of cartridges fitting into the pen device used by the patient who may then avoid the reconstitution of the preparation and, hence, will not have to be in the possession of a lyophilized preparation, a suitable vehicle for reconstitution as well as the necessary skill and sterile equipment for sterile reconstitution of the preparation.

For safety reasons it will also be desirable to avoid the reconstitution of a lyophilized preparation just before the use of the preparation.

Furthermore, it would also be an advantage to avoid the lyophilization step in the production of growth hormone preparations. Lyophilization is a time consuming and costly process and is also often a "bottleneck" in the production due to the limited capacity of the freeze drier.

Thus, there is a need to reduce the rate of the degradation processes in order to allow for dissolved hGH preparations being stable during shelf life and during the period of use of up to one month.

Prior attempts to stabilize hGH have not fully succeeded in preventing the formation of dimer. The problems associated with dimer formation is e.g noted in Becker, G.W., Biotechnology and Applied Biochemistry 9, 478 (1987).

International Patent Publication No. WO 89/09614 and Australian patent application No. 30771/89 disclose a stable pharmaceutical formulation containing human growth hormone, glycine, and mannitol. Such a preparation shows improved stability during normal processing and storage in a lyophilized state as well as in the period of use after the reconstitution.

Published European patent application No. 303 746 discloses that animal growth hormone may be stabilized with various stabilizers to give decreased formation of insolubles and preservation of the soluble activity in aqueous environments, such stabilizers including certain polyols, amino acids, polymers of amino acids having a charged side group at physiological pH, and choline salts. Polyols are selected from the group consisting of non-reducing sugars, sugar alcohols, sugar acids, pentaerythritol, lactose, water-soluble dextrans and Ficoll; amino acids are selected from the group consisting of glycine, sarcosine, lysine or salts thereof, serine, arginine or salts thereof, betaine, N,N,-dimethyl-glycine, aspartic acid or salts thereof, glutamic acid or salts thereof; a polymer of an amino acid having a charged side group at physiological pH may be selected from polylysine, polyaspartic acid, polyglutamic acid, polyarginine, polyhistidine, polyornithine and salts thereof; and choline derivatives are selected from the group consisting of choline chloride, choline dihydrogen citrate, choline bitartrate, choline bicarbonate, tricholine citrate, choline ascorbate, choline borate, choline gluconate, choline phosphate, di(choline)sulphate and dicholine mucate.

EP 374120 discloses a stabilized preparation of growth hormone comprising a buffered polyol excipient comprising a polyol having three hydroxy groups and a buffer to achieve a pH in a range in which the growth hormone retains its bioactivity for a sufficient period of time. Histidine is mentioned as a buffer for a polyol having three hydroxy groups.

US 4,917,685 discloses a stabilized growth promoting formulation comprising a growth hormone, and a stabilizer selected among certain polyols, amino acids and choline derivatives. The formulation may be in the form of an aqueous solution.

US 4,815,568 discloses a stabilized growth promoting formulation comprising a growth hormone, and a stabilizer selected among certain amino acids, amino acid polymers and choline derivatives. The formulation may be in the form of an aqueous solution.

JP-A-63115821 discloses a powdery composition containing growth hormone, a basic amino acid (e.g. histidine) and/or a salt thereof. The composition is intended for nasotracheal administration.

### DESCRIPTION OF THE INVENTION

It has now surprisingly been found that a preparation of a human growth hormone derivative comprising histidine as additive or buffering substance in an amount of from 0.1 to 12 mg histidine or derivative thereof per mg of growth hormone derivative shows a very high stability against deamidation, oxidation and cleavage of peptide bonds. The stability of the product allows for the storing and shipment thereof in the form of a dissolved preparation.

EP 303746 mentions polyhistidine as a potential stabilizer for animal growth hormone but there is no indication whether it stabilizes an animal growth hormone or human growth hormone.

EP 374120 teaches that histidine hydrochloride may be used as a buffer for buffering a polyol having three hydroxy groups for improving the stability of a growth hormone preparation in the form of a solution comprising a high concentration of growth hormone and a polyol as stabilizer. Histidine hydrochloride must be added in an amount of about 3% by weight of the solution corresponding to a concentration of -0.15 M solution of histidine hydrochloride. EP 374120 also teaches that histidine alone does not impart chemical and physical stability to a growth hormone preparation.

The preparation of the invention is a pharmaceutical preparation of a human growth hormone derivative in the form of a buffered aqueous solution of the growth hormone derivative buffered with histidine buffer and having a pH of from 5 to 8. Histidine is present in the preparation in an amount of from 0.1 to 12 mg histidine per mg of hGH derivative. Such preparation is in a ready-to-use form and may be stored and shipped as an aqueous solution without any considerable degradation. L-histidine has a pKA of 6.0 and is, accordingly suitable as a buffer itself in the pH interval in question.

Preparations having a pH from 6 to 7.5 are preferred.

In pharmaceutical preparations of the invention, the concentration of histidine is preferably from 1 mM to 100 mM. More preferred, the concentration of histidine is from 2 to 20 mM.

The growth hormone derivative in pharmaceutical preparations of the invention is selected from: truncated forms of human growth hormone wherein one or more amino acid residues present in hGH has (have) been deleted; analogues of hGH wherein one or more amino acid residues in the native molecule has (have) been substituted by another amino acid residue (preferably the residue of a naturally occurring amino acid, as long as the substitution does not have any adverse effect, such as antigenicity or reduced action); and N- or C-terminally extended forms of hGH (such as Met-hGH, Met-Glu-Ala-Glu-hGH or Ala-Glu-hGH)

The content of histidine in the preparations of the invention is calculated using the molar weight of histidine itself.

In the present context "high stability" is obtained when the preparation is more stable than conventional formulations comprising phosphate buffer.

### EXPERIMENTAL PART

The examples below, which relate to hGH per se, are included to illustrate the stabilising effect of histidine (His) on the hGH polypeptide chain in aqueous solution.

### EXAMPLE 1.

### Reduction of the deamidation.

The rate of deamidation was examined at 37°C for hGH preparations comprising 4IU and 12IU at pH 6.5 in His buffer as compared to phosphate buffer at the same pH.

The hGH preparation comprising 4 IU having the composition A was prepared by dissolving 13.3 mg biosynthetic hGH in 10 ml 10 mM histidine buffer prepared by dissolving 15.5 mg histidine in 10 ml deionized water containing 0.9% benzyl alcohol and adding 0.1 N hydrochloric acid to pH 6.5. The preparation comprising 12 IU was prepared by dissolving 40 mg hGH in the same constituents as stated above.

The hGH preparation comprising 4IU having the composition B was prepared by dissolving 13.3 mg biosynthetic hGH in 10 ml 10 mM disodium phosphate prepared by dissolving 17.8 mg disodium-hydrogen-phosphate in 10 ml deionized water, containing 0.9% (v/v) of benzyl alcohol and adding 0.1 N phosphoric acid to pH 6.5. The preparation comprising 12 IU was prepared by dissolving 40 mg hGH in the same constituents as stated above.

### Composition A:

10 mM His
0.9% benzyl alcohol
HCl ad pH 6.5

### Composition B:

10 mM disodium phosphate
0.9% benzyl alcohol
phosphoric acid ad pH 6.5

The preparations were examined by IE-HPLC for the contents of desamido-hGH immediately after the reconstitution and after 7 days at 37°C. The results appear from the below Table 1.

**TABLE 1.**

| Deamidation. | | |
|---|---|---|
| | Preparation | Desamido |
| | % | |
| Buffer A | 4IU/ml | 1.7 |
| start | 12 IU/ml | 2.1 |
| Buffer A | 4IU/ml | 10.1 |
| 7 days at 37 °C | 12IU/ml | 10.4 |
| Buffer B | 4 UI/ml | 1.8 |
| Start | 12 IU/ml | 2.3 |
| Buffer B | 4 UI/ml | 16.9 |
| 7 days at 37°C | 12 IU/ml | 14.9 |

From the above figures it appears that the deamidation of hGH is significantly reduced at 37°C in histidine buffer as compared with phosphate buffer.

### EXAMPLE 2:

### Reduction of the deamidation in the presence of histidine or histidine derivatives.

The rate of deamidation was examined at 25°C for hGH preparations comprising 6 IU hGH at pH 6.5 and at pH 7.3 in 5 mM, 10 mM and 100mM His buffer as compared to 8 mM phosphate buffer at the same pH. Furthermore, the histidine derivatives His-Gly, His-Ala, His-Leu, His-Lys, His-Phe, His-Ser, Histidine methyl ester, histidinol, imidazol, imidazol-4-acetic acid, and histamine were tested.

The hGH preparations were prepared by dissolving 20 mg biosynthetic hGH in 10 ml of histidine buffer of the desired strength prepared by dissolving 7.8 mg, 15.5 mg, and 155.2 mg, respectively, of histidine in 10 ml deionized water containing 0.9% (v/v) of benzyl alcohol and adding 0.1 N hydrochloric acid to the stated pH.

The hGH formulations stated in the below Table 2 were stored at 25°C and analyzed for the desamido contents after 14 and 30 days by IE-HPLC. The results appear from the below Table 2.

**Table 2.**

| Contents of desamido hGH as determined by IE-HPLC as a function of the formulation and the time in solution at 25°C: | | |
|---|---|---|
| Formulation (*) | Formation of desamido compound **at 25°C** | |
| | **14 days (')** | **30 days** |
| 5 mM His pH 6.5 | 6.5 | 9.1 |
| 5 mM His pH 7.3 | 11.0 | 17.4 |
| 10 mM His pH 6.5 | 6.8 | 9.7 |
| 10 mM His pH 7.3 | 11.3 | 16.6 |
| 100 mM His pH 6.5 | 9.8 | 15.2 |
| 100 mM His pH 7.3 | 19.3 | 28.8 |
| 8 mM di-Na-phosphate | 7.8 | 10.8 |
| pH 6.5 | | |
| 8 mM di-Na-phosphate | 15.2 | 20.3 |
| pH 7.3 | | |
| 8 mM di-Na-phosphate | 9.4 | 13.2 |
| pH 6.5, | | |
| 0.3% m-cresol | | |
| 10 mM Asp, pH 6.5 | 21.7 | nd |
| 10 mM Glu, pH 6.5 | 14.8 | nd |
| 10 mM His-Gly, | 5.6 | 8.1 |
| pH 6.2 | | |
| 10 mM His-Ala | 6.2 | 8.5 |
| pH 6.5 | | |
| 10 mM His-Leu | 8.8 | 12.3 |
| pH 6.5 | | |
| 10 mM His-Lys | 8.6 | 12.0 |
| pH 6.5 | | |
| 10 mM His-Phe | 7.5 | 11.3 |
| pH 6.5 | | |
| 10 mM His-Ser | 22.0 | nd |
| pH 6.3 | | |
| 10 mM His-methyl- | 4.6 | 5.2 |
| ester,pH 6.5 | | |
| 10 mM histidinol | 27.4 | nd |
| pH 6.5 | | |
| 10 mM imidazole | 9.2 | 12.2 |
| pH 6.5 | | |
| 10 mM imidazol- | 10.3 | 14.2 |
| 4-acetic acid | | |
| pH 6.5 | | |
| 10 mM Histamine | 9.8 | 12.2 |
| pH 6.5 | | |

| | | |
|---|---|---|
| *: Comprises 0.9% benzyl alcohol, except formualtion No.9 The contents of desamido-hGH in starting material was: 2.1 % | | |

From the above Table 2 it appears that the de-amidation of hGH is reduced by approximately 20% by the addition of histidine as compared with phosphate buffer at ph 6.5 and 7.3. Furthermore, a reduction of the pH from 7.3 being the conventional pH of commercial hGH preparations to 6.5 in itself gives rise to a reduction of the rate of de-amidation by 50%.

Histidinol does not seem to stabilize the preparations under the test conditions, and addition of histidine in larger amounts does not add but rather detract from the desired effect.

Comparable results are seen to be obtained using histidine analogues such as imidazole, histamine, and imidazol-4-acetic acid as well as the histidine methyl ester giving rise to the formation of only 3.1% desamido-hGH after 30 days at 25°C, allowing for a life-time of the preparation of 3-4 months.

Addition of Asp or Glu increases the rate of deamidation as compare to phosphate at pH 6.5.

Addition of dipeptides of the type His-X shows positive effect for His-Gly and His-Ala, whereas His-Ser reduces the stability to de-amidation.

The above results show that the rate of de-amidation is reduced by lowering the pH and by adding histidine in a low concentration, preferably about 5mM-10mM. The rate of de-amidation may be reduced by more than 50% by lowering the pH and substituting the phosphate buffer with histidine.

The use of m-cresol or benzyl alcohol as preservative seems to have no influence on the rate of de-amidation.

Split-formation (hydrolysis of peptide bonds) is reduced by histidine at pH 6.5 in comparison with phosphate.

### EXAMPLE 3.

### Reduction of the formation of sulfoxide.

The dependency og the pH and the type of buffer was examined.

### Dependency of pH:

### Formulation:

A commercial hGH preparation (Norditropin^{®}, 12 IU/ml) comprising bicarbonate, glycine and mannitol + 0.9% benzyl alcohol was adjusted to pH 8.3, 8.0, 7.5, 7.0, 6.5 and 6.0 using 0.1 N hydrochloric acid, and the samples were left at 37°C. Analysis was carried out by RP-HPLC after 0, 7 and 14 days. The results appear from the below Table 3.

**Table 3.**

| **Formation of Sulfoxide** | | | |
|---|---|---|---|
| Sample | Temp °C | Days | Sulfoxide % |
| pH 8.37 | - | 0 | 1.0 |
| pH 8.37 | 37 | 7 | 9.0 |
| pH 8.04 | 37 | 7 | 8.7 |
| pH 7.52 | 37 | 7 | 8.3 |
| pH 7.01 | 37 | 7 | 7.7 |
| pH 6.52 | 37 | 7 | 6.5 |
| pH 6.02 | 37 | 7 | 4.8 |
| pH 8.37 | 37 | 14 | 14.9 |
| pH 8.04 | 37 | 14 | 14.5 |
| pH 7.52 | 37 | 14 | 14.0 |
| pH 7.01 | 37 | 14 | 12.9 |
| pH 6.52 | 37 | 14 | 11.1 |
| pH 6.02 | 37 | 14 | 7.7 |

As will be appreciated, the formation of sulfoxide of hGH is reduced when lowering the pH from 8.4 to 6.0.

### Type of Buffer, pH:

A B-hGH preparation comprising 12 mg/ml distilled water was diluted in the proportion 1+10 with various buffers in a concentration of 15 mM and optional added further additive(s). The samples were left at 25°C, and analysis by RP-HPLC was carried out after 10 and 34 days. The results of the RP-HPLC and optional additives appear from the below Table 4.

**Table 4.**

| Formation of Sulfoxide | | | | |
|---|---|---|---|---|
| Buffer | pH | Additive | Sulfoxidated | |
| | | | 10d | 34d |
| | | | % | |
| Phosphate | 7.3 | - | 1.9 | 5.5 |
| Histidine | 7.3 | - | 0.9 | 2.4 |
| Histidine | 6.9 | - | 0.9 | 2.0 |
| Histidine | 6.5 | - | 0.8 | 1.9 |
| Histidine | 7.3 | 18 mM Met | 0.8 | 2.0 |
| Histidine | 7.3 | 18 mM Cys | 2.4 | 2.9 |
| Histidine | 7.3 | 0.42mM toc. | 1.1 | 3.0 |
| Histidine | 7.3 | 9% ethanol | 1.3 | 4.2 |
| Histidine | 7.3 | 18 mM asc. | 41 | nd |
| Histidine | 7.3 | 0.8% NaCl | 1.3 | 3.5 |

| | | | | |
|---|---|---|---|---|
| Toc.: tocoferol; asc.: ascorbic acid. | | | | |

As compared with phosphate buffer, a marked reduction of the formation of sulfoxidated B-hGH is observed in histidine buffer (pH 7.3). A reduction of the formation of sulfoxide is observed with falling pH in His-buffer.

No further effect was obtained by addition of anti-oxidants or other additives.

### EXAMPLE 4

In an analogous manner as described in EXAMPLE 1, biosynthetic human growth hormone was formulated in a concentration of 6 IU/ml in 0.9% benzyl alkohol at pH 6.5 in various concentrations of histidine, 0, 1, 2, 5, 10, 20, 30, 50, or 100 mM.

The samples were stored 7 days at 37°C and analysed for the contents of desamido, oxidized forms and dimers and polymers were performed in the same manner as described above. The results appear from the below Table 5 wherein the contents of desamido-hGH, dimers and polymers, and oxidized forms are determined by IE-HPLC, GP-HPLC and RP-HPLC and the contents of the cleaved forms of hGH is measured by IE-HPLC.

The amount of Dimer is low when the concentration is 1 mM histidine or above, for formation of desamido compounds concentrations of histidine of up to 30 mM gives acceptable results, and for formation of oxidized forms concentrations of histidine below 20 mM are preferred. An overall optimum is seen for a concentration of histidine of 5 mM.

**Table 5**

| Concentrat. of Histidine mM | Amount of Desamido in % | Amount of Dimer in % | Amount of Polymers in % | Amount of Oxidized forms in % | Cleaved Forms in % | pH |
|---|---|---|---|---|---|---|
| 0 | 7.7 | 0.7 | <0.2 | 1.5 | 1.4 | 6.3 |
| 1 | 7.9 | 0.4 | <0.2 | 1.5 | 1.2 | 6.4 |
| 2 | 8.7 | 0.3 | <0.2 | 1.6 | 1.2 | 6.5 |
| 5 | 9.6 | 0.4 | <0.2 | 1.7 | n.d. | 6.6 |
| 10 | 10.8 | 0.4 | <0.2 | 1.7 | n.d. | 6.6 |
| 20 | 11.6 | 0.4 | <0.2 | 2.0 | n.d. | 6.6 |
| 30 | 12.0 | 0.3 | <0.2 | 2.1 | n.d. | 6.6 |
| 50 | 14.4 | 0.5 | <0.2 | 3.8 | n.d. | 6.6 |
| 100 | 17.0 | 0.3 | <0.2 | 2.6 | n.d. | 6.6 |

| | | | | | | |
|---|---|---|---|---|---|---|
| n.d. not detectable | | | | | | |

### REFERENCES

1) Y.-C.J. Wang and M.A. Hanson. Parenteral Formulations of Proteins and Peptides: Stability and Stabilizers. J. Parenteral Science and Technology 42 (Suppl.) (1988) 53-525.
2) M.C. Manning, K. Patel, R.T. Borchardt. Stability of Protein Pharmaceuticals. Pharmaceutical Research 6 (11) (1989) 903-918.
3) B.A. Johnson, J.M. Shirokawa, W.S. Hancock, M.W. Spellman, L.J. Basa and D.W. Asward. J.Biol.Chem. 264, 1462-71 (1989).
4) L.C. Teh et al., J.Biol.Chem., 262, 785-794 (1987).
5) G.W. Becker et al., Biotech.Appl.Biochem., 10, 326-337 (1988).
6) R.A. Houghten et al., Arch.Biochem.Biophys., 178, 350-355 (1977).
7) R.M. Riggin et al., Anal.Biochem., 167, 199-209 (1987).
8) P. Gellerfors et al., Acta Paediatr.Scand (suppl), 370, 93-100 (1990).
9) M.J. Kaufman, Pharm.Res., 7 (3) 289-292 (1990).

## Claims

1. A pharmaceutical preparation in the form of a buffered aqueous solution of a human growth hormone derivative having a pH of from 5 to 8, and comprising:
a derivative of human growth hormone (hGH) selected from:
truncated forms of hGH wherein one or more amino acid residues present in hGH have been deleted;
analogues of hGH wherein one or more amino acid residues present in hGH are substituted by another amino acid residue; and
N- or C-terminally extended forms of hGH;
and
histidine in an amount of from 0.1 to 12 mg histidine per mg of hGH derivative.

2. A pharmaceutical preparation according to claim 1, wherein histidine is present in a concentration of from 1 mM to 100 mM.

3. A pharmaceutical preparation according to claim 1 or 2, wherein histidine is present in a concentration of from 2 mM to 20 mM.

4. A pharmaceutical preparation according to any one of claims 1-3, having a pH of from 6 to 7.5.

## Patentansprüche

1. Arzneimittel in der Form einer gepufferten wässrigen Lösung eines Humanwachstumshormonderivats mit einem pH-Wert von 5 bis 8, und umfassend:
ein Derivat von Humanwachstumshormon (hGH), ausgewählt aus:
verkürzten Formen von hGH, wobei ein oder mehrere in hGH vorliegende Aminosäurereste deletiert worden sind;
Analoga von hGH, wobei ein oder mehrere in hGH vorliegende Aminosäurereste durch einen anderen Aminosäurerest substituiert sind; und
N- oder C-terminal verlängerte Formen von hGH;
und
Histidin in einer Menge von 0,1 bis 12 mg Histidin pro mg hGH-Derivat.

2. Arzneimittel nach Anspruch 1, wobei Histidin in einer Konzentration von 1 mM bis 100 mM vorliegt.

3. Arzneimittel nach Anspruch 1 oder 2, wobei Histidin in einer Konzentration von 2 mM bis 20 mM vorliegt.

4. Arzneimittel nach einem der Ansprüche 1-3 mit einem pH-Wert von 6 bis 7,5.

## Revendications

1. Une préparation pharmaceutique sous forme d'une solution aqueuse tamponnée d'un dérivé d'hormone de croissance humaine ayant un pH de 5 à 8 et comprenant :
un dérivé d'hormone de croissance humaine (hGH) choisi parmi :
les formes tronquées de hGH d'où ont été supprimés un ou plusieurs résidus d'acides aminés présent dans hGH ;
les analogues de hGH où un ou plusieurs résidus d'acides aminés présent dans hGH sont substitués par un autre résidu d'acide aminé ; et
les formes de hGH à extension terminale N- ou C- ;
et
de l'histidine en une quantité de 0,1 à 12 mg d'histidine par mg de dérivé de hGH.

2. Une préparation pharmaceutique selon la revendication 1, où l'histidine est présente à une concentration de 1 mM à 100 mM.

3. Une préparation pharmaceutique selon la revendication 1 ou 2, où l'histidine est présente à une concentration de 2 mM à 20 mM.

4. Une préparation pharmaceutique selon l'une des revendications 1 à 3, ayant un pH de 6 à 7,5.
